(19) Europäisches Patentamt / European Patent Office / Office européen des brevets

(11) **EP 1 682 107 B1**

(12) **EUROPEAN PATENT SPECIFICATION**

(45) Date of publication and mention of the grant of the patent: **12.08.2009 Bulletin 2009/33**

(21) Application number: **04798492.7**

(22) Date of filing: **12.11.2004**

(51) Int Cl.:
***A61K 31/01*** *(2006.01)* ***A61K 31/015*** *(2006.01)*
***A61P 35/00*** *(2006.01)*

(86) International application number: **PCT/GB2004/004769**

(87) International publication number: **WO 2005/049001 (02.06.2005 Gazette 2005/22)**

(54) **ANTI-TUMOUR TERPENE COMPOUNDS**

ANTITUMORALE TERPEN-VERBINDUNGEN

COMPOSES TERPENIQUES ANTITUMORAUX

(84) Designated Contracting States:
**AT BE BG CH CY CZ DE DK EE ES FI FR GB GR HU IE IS IT LI LU MC NL PL PT RO SE SI SK TR**

(30) Priority: **13.11.2003 GB 0326518**

(43) Date of publication of application: **26.07.2006 Bulletin 2006/30**

(73) Proprietor: **Compton Developments, Ltd.**
**Swansea SA6 8YH (GB)**

(72) Inventors:
- **BOWEN, Ifor, Delme,**
  **Cardiff University**
  **Cardiff CF10 3US (GB)**
- **ALI, Ahmed, Yasine,**
  **Cardiff University**
  **Cardiff CF10 3US (GB)**

(74) Representative: **Tombling, Adrian George et al**
**Withers & Rogers LLP**
**Goldings House**
**2 Hays Lane**
**London**
**SE1 2HW (GB)**

(56) References cited:
**WO-A1-01/80868**
**WO-A1-02/07744**
**WO-A1-94/20080**
**WO-A1-02/080951**
**WO-A2-02/053138**
**WO-A2-20/04066912**

- **TATMAN D ET AL: "VOLATILE ISOPRENOID CONSTITUENTS OF FRUITS, VEGETABLES AND HERBS CUMULATIVELY SUPPRESS THE PROLIFERATION OF MURINE B16 MELANOMA AND HUMAN HL-60 LEUKEMIA CELLS" CANCER LETTERS, NEW YORK, NY, US, vol. 175, no. 2, 2002, pages 129-139, XP008037328 ISSN: 0304-3835 cited in the application**
- **QURESHI S ET AL: "EVALUATION OF THE GENOTOXIC, CYTOTOXIC, AND ANTITUMOR PROPERTIES OFCOMMIPHORA MOLMOL USING NORMAL AND EHRLICH ASCITES CARCINOMA CELL-BEARING SWISS ALBINO MICE" CANCER CHEMOTHERAPY AND PHARMACOLOGY, SPRINGER VERLAG, BERLIN, DE, vol. 33, no. 2, 1993, pages 130-138, XP001035052 ISSN: 0344-5704 cited in the application**
- **AL-HARBI M M ET AL: "ANTICARCINOGENIC EFFECT OF COMMIPHORA MOLMOL ON SOLID TUMORS INDUCED BY EHRLICH CARCINOMA CELLS IN MICE" CHEMOTHERAPY, S. KARGER, BASEL, CH, vol. 40, no. 5, September 1994 (1994-09), pages 337-347, XP001035099 ISSN: 0009-3157 cited in the application**
- **SALEH M M ET AL: "Cytotoxicity and in vitro effects on human cancer cell lines of volatiles of Apium graveolens var. filicum" PHARMACEUTICAL AND PHARMACOLOGICAL LETTERS, vol. 8, no. 2, April 1998 (1998-04), pages 97-99, XP008041812 ISSN: 0939-9488**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 682 107 B1

- **LEAL PATRICIA F ET AL: "Functional properties of spice extracts obtained via supercritical fluid extraction." JOURNAL OF AGRICULTURAL AND FOOD CHEMISTRY, vol. 51, no. 9, 23 April 2003 (2003-04-23), pages 2520-2525, XP008041804 ISSN: 0021-8561 cited in the application**
- **'Gummi Myrrha' WHO MONOGRAPHS ON SELECTED PLANTS pages 247 - 256**
- **BASER K.H.C. ET AL FLAVOUR AND FRAGRANCE JOURNAL vol. 18, 2003, pages 153 - 156**
- **DEKEBO A. ET AL FITOTERAPIA vol. 73, 2002, pages 48 - 55**
- **HANUS L.O. BIOMED. PAPERS vol. 149, no. 1, 2005, pages 3 - 28**
- **THULIN M. ET AL ECONOMIC BOTANY vol. 45, no. 4, 1991, pages 487 - 494**


Remarks:

The file contains technical information submitted after the application was filed and not included in this specification

## Description

The present invention relates to the use of a terpene compound in the treatment or prophylaxis of cancer. The present invention also relates to a pharmaceutical composition comprising the terpene compound and a pharmaceutically acceptable excipient, carrier, adjuvant or vehicle.

Myrrh, the resinous product of the African shrub, *Commiphora molmol*, has a long folk history of healing. The medicinal background of Myrrh and scented Myrrh (*Commiphora guidotti*, or "Haddi" (Somali name), or "Opoponax" (Western common name)) is largely based on ancient folklore and has found general use in Arab, Indian and Chinese herbal remedies. In this context it is known to be non-toxic and to generally exhibit anti-inflammatory, antiseptic and healing properties. It is also widely used in the perfume and flavouring industries. Its specific anti-tumour properties have not been systematically or scientifically tested, although there has been a preliminary report of the activity of the crude extract of *Commiphora molmol* against Ehrlich solid tumour in mice (Al Harbi et al., Experimental Chemotherapy, 40, 337-347, 1994). The crude extract is also cited in a number of herbal remedies for use in immunological disorders and against *Schistosomiasis*.

The activity of individual chemical components of *C. molmol* and *C. gudiotti* has not been analysed. Our initial chemical analysis of extracts shows them to be a very rich source, comprising of some 300 compounds.

In terms of background information, the use of Myrrh in many herbal remedies is known but no prior art documents specify the systematic identification of specific anticancer chemical compounds from Myrrh. A few references indicate the efficacy of a crude extract of Myrrh (*Commiphora molmol)* against a mouse tumour (Qureshi & Al Harbi et al., Cancer Chemotherapy and Pharmacology, 33, 130-138, 1993; and Al-Harbi *et al.*, 1994 (*supra*)).

International Patent Application WO 00/50053 describes the use of Myrrh (*Commiphora molmol*) with other plants as a classical herbal treatment of "cancer", again using crude extracts. A further publication (Rao et al., J. Ethnopharmacology, 76, 151-154, 2001) describes the non toxic impact of *Commiphora molmol* on mice.

Tatman et al., (Cancer Letters, 175, 129-139, 2002) discloses that volatile isoprenoids are active against murine melanoma and human leukaemia cell lines.

Leal et al., (J. Agric. Food. Chem., 51, 2520-2525, 2003) discloses that ginger and tumeric extracts showed anti-cancer activities.

Saleh et al., (Pharm. Pharmacol. Lett., 8, 97-99, 1998) discloses that volatiles of celery are cytotoxic against human cancer cell lines.

US patent US-A-6,486,706 discloses the use of $\alpha$- and $\beta$-santatols in the treatment of viral-induced tumours.

US patent US-A-5,602,184 discloses the use of selected terpenes against human cancer cell lines.

US patent application US 2002/0155522 discloses the use of $\beta$-elemene against tumour cells.

International PCT application WO 01/80868 disclose the use of sesquiterpene mixtures for preventing and treating cancers.

Itokawa et al., (Chem. Pharm. Bull., 33, 3488-3492, 1985) describes studies on the anti-tumour activity of bisabolene sesquiterpenoids.

A range of compounds obtained from *C. molmol* and/or *C. gudiotti* can be gauged from the spectra or peaks of chemicals obtained by GC/MS and HPLC/MS. These peaks can be sampled giving a wide range of new molecules to be tested. Some of the innovative aspects of the work include: 1) choosing the biomedically rich source of untested molecules in Myrrh (*Commiphora molmol*) and Haddi or scented Myrrh (*Commiphora guidotti*), 2) the identification and characterisation of a wide source of novel compounds from these Myrrh extracts, and 3) the use of specific (apoptotic) bioassays to select non-toxic compounds suitable for controlling human tumour cell lines.

None of these prior art documents discloses any specific compound from Myrrh or scented Myrrh having anti-cancer activity against human tumours. As will be appreciated, there is demand for effective anti-cancer drugs and in particular for drugs that have no, or limited, toxicity against non-cancerous cells. The inventors have identified a number of specific compounds that specifically induce apoptosis in human tumour cells but not in normal tissues.

Current treatments using drugs such as Taxol, Paclitaxel and Vinblastin result in a damaging form of inflammatory cell death or necrosis. Drugs selected for apoptosis, which is a natural process of programmed cell death, should induce little or no side effects. This approach is innovative in that it selects for a benign mechanism of action that should be attractive to both the pharmaceutical industry and ultimately to the medical profession. This selective approach has revealed non-toxic anticancer agents.

According to a first aspect of the present invention there is provided the use of a compound isolated from a plant, or synthesised, in the manufacture of a medicament for the treatment or prophylaxis of cancer, wherein the compound is selected from:

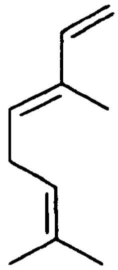

trans-β-Ocimene; and

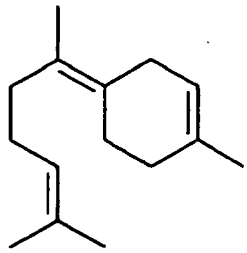

γ-bisabolene

It has been found that the specific terpene compounds referred to above act as anti-cancer agents by inducing apoptosis of human cancer cells.

Preferably the compound is trans-β-ocimene.

In other embodiments, the compound is γ-bisabolene.

The compound used in the first aspect of the present invention can be obtained from a plant in the genus *Commiphora*. In particular, it is preferred that the compound can be obtained from the plant *Commiphora guidotti.*

The compound used in the first aspect of the present invention can be obtained by isolating it from the appropriate plant indicated above. In particular, the compounds can be isolated from *Commiphora guidotti.* The compound can be isolated using any suitable preparative method. Methods of isolating such compounds are known to those skilled in the art and include chromatographic methods such as flash column chromatography and solid phase extraction columns. Nuclear Magnetic Resonance and Mass Spectrometry can be used to identify the individual compounds.

When the compound used in the composition of the present invention is isolated from a plant, substantially no contaminating plant material is present. Substantially no contaminating plant material means that less than 0.1 % (w/w) of contaminating plant material is present.

The compound used in the composition of the present invention may be obtained from commercial sources such as RC Treat Ltd (*trans*-β-Ocimene and γ-bisabolene). The compound of the present invention may also be synthesised using standard chemical synthesis procedures.

Two or more of the terpene compounds may be used together as an anti-cancer agent. Furthermore, additional anti-cancer agents, therapeutics, markers, etc. can also be used in conjunction with the terpene compounds used according to the first aspect of the present invention.

The term "anti-cancer" agent refers to an agent that can be used to prevent or reduce the development of a cancer or that can reduce the size of a cancer.

The cancer to be prevented or treated according to the first aspect of the present invention can be any cancer, but is preferably a human cancer. Preferred cancers include breast cancer, ovarian cancer, prostate cancer, colon cancer, lung cancer, pancreatic cancer, bowel cancer, melanoma, testicular cancer, cervical cancer, fibrosarcoma, squamous cell carcinoma, leukaemia, astrocytoma and glioma.

It has been found that the compound used in the first aspect of the present invention produces a differentially higher level of apoptosis in cancer cells than in corresponding normal cells. This means that at suitable doses the compounds are differentially active against cancer cells.

According to a second aspect of the present invention there is provided a pharmaceutical composition comprising trans-β-Ocimene isolated from a plant, or synthesised, in combination with a pharmaceutically acceptable excipient, carrier, adjuvant or vehicle.

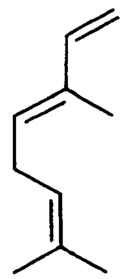

trans-β-Ocimene

The terpene compound can be delivered to an individual in combination with any pharmaceutically acceptable carrier, adjuvant or vehicle. Pharmaceutically acceptable carriers, adjuvants and vehicles that may be used include, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protomine sulphate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene block polymers and wool fat.

The pharmaceutical composition may be administered orally, parentally, by inhalation spray, topically, rectally, nasally, buccally, vaginally or by an implanted reservoir. Preferably, the pharmaceutical composition is administered by injection. The term "parenteral" as used herein includes subcutaneous, intracutaneous, intravenous, intramuscular, intra-articular, intrasynovial, intrasternal, intrathecal, intralesional and intracranial injection or infusion techniques.

The pharmaceutical composition may be delivered in the form of a sterile injectable preparation, for example as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parentally-acceptable diluent or solvent, for example as a solution in 1, 3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or di glycerides. Fatty acids such as oleic acid and its glyceride derivatives are useful in the preparation of injectables, as are naturally pharmaceutically acceptable oils such as olive oil or caster oil, especially in their polyoxyethyated versions. These oil solutions or suspensions may also contain a long chain alcohol diluent or dispersant such as Ph. Helv or a similar alcohol.

The pharmaceutical composition of the present invention may also be administered as a fluid or in the form of suppositories for rectal administration. The suppository can be prepared by terpene compound with a suitable non-irritating excipient which is solid at room temperature but liquid at the rectal temperature and therefore will melt in the rectum to release the terpene compound. Such materials include cocoa butter, beeswax and polyethylene glycols.

Topical administration of the composition may be desirable when the desired treatment involves areas or organs readily accessible for topical application. For application topically to the skin, the terpene compound should be formulated with carriers for topical administration, such as, mineral oil, liquid petroleum, white petroleum, propylene glycol, polyoxyethylene, polyoxypropylene compounds, emulsifying wax and water. Alternatively, the terpene compound can be formulated with a suitable lotion or cream, or dissolved in a carrier. Suitable carriers include mineral oil, sorbitan monosterate, polysorbate 60, cetyl esters, wax, cetearyl alcohol, 2-octyldodecanol, benzyl alcohol and water. The terpene compound can be applied topically to the lower intestinal tract by a rectal suppository formulation or as a suitable enema formulation.

The pharmaceutical composition of the present invention may be administered by nasal aerosol or inhalation. Suitable compositions for such administration can be prepared according to techniques well known to those skilled in the art of pharmaceutical formulation and can be prepared as solutions in saline, employing benzyl alcohol or other preservatives, absorption promoters to enhance bio-availability, fluorocarbons, and/or other solublising other dispersing agents known in the art.

The precise amount of the terpene compound to be delivered to a patient will depend upon the severity of the

cancer state, the general health, age, weight and gender of the subject, diet, time and frequency of administration, reaction sensitivities, and tolerance/response to therapy. A suitable amount can be determined by routine experimentation and is within the judgement of the clinician. Generally, and effective dose will be from 0.2μM to 100mM, more preferably 0.2μM to 10μM.

The pharmaceutical composition of the present invention may be administered in combination with other drugs, agents or markers.

The present invention also provides the use of a *Commiphora guidotti* extract in the manufacture of a medicament for the treatment or prophylaxis of cancer.

The *Commiphora guidotti* extract can be any extract and preferably it contains at least one of the compounds used in the first aspect of the present invention. Preferably the extract contains at least one of trans-β-ocimene, γ-bisabolene and α-bisabolene.

The extract is preferably an alcoholic extract and may be obtained using standard procedures for obtaining alcoholic extracts of the plant. In particular, methods for obtaining such an alcoholic extract are well known to those skilled in the art. Alternatively, it is preferred that the plant material is an alcoholic extract of an essential oil of the plant. Essential oils are the volatile, organic constituents of fragment plant matter. Essential oils are generally extracted from plant by two main methods, distillation (steam, water or dry distillation) and cold pressing. A plant extract containing mainly oils can also be prepared using solvents, carbon dioxide extraction or hydrofluoroalkanes. Tincture plant extracts can be made by macerating the plant materials and extracting using aqueous, ethanolic solvents (70%-90% ethanol in water) and left for a period of time, after which the solid debris is filtered.

The following examples, with reference to the figures, are offered by way of illustration. However, C. molmol extracts do not form part of the invention.

Figure 1 shows the growth curve of human fibrosarcoma cells with and without addition of 667ug/ml *C. molmol* and Haddi extracts to the growth medium.

Figure 2 shows growth curve of S 180 fibrosarcoma cells with and without the addition of 667ug/ml *C. molmol* and Haddi extracts to the growth medium.

Figure 3 shows the cytotoxic effects of ethanol extracted *C. molmol* on S180 fibrosarcoma cell lines.

Figure 4 shows the cytotoxic effects of ethanol extracted *C. molmol* on HT1080 human fibrosarcoma cell lines.

Figure 5 shows the cytotoxic effects of hexane extracted Haddi on S180 fibrosarcoma cell lines.

Figure 6 shows the cytotoxic effects of hexane extracted Haddi on HT1080 human fibrosarcoma cell lines.

Figure 7 shows the cytotoxic effects of *trans*-β-Ocimene S180 tumour cell lines.

Figure 8 shows the cytotoxic effects of *trans*-β-Ocimene on HT1080 tumour cell lines.

Figure 9 shows the cytotoxic effects of γ-Bisabolene on S180 tumour cell lines.

Figure 10 shows the cytotoxic effects of γ-Bisabolene on HT1080 tumour cell lines.

Figure 11 shows the induction of apoptotic cell death in HT1080 human fibrosarcoma cells treated with 5uM Bisabolene over 12 hours.

Figure 12 shows the effect of ethanolic extracted *C. molmol* on normal fibroblast cells.

Figure 13 shows the effect of hexane extracted Haddi on normal fibroblast cells.

Figure 14 shows a mixed tissue culture treated for 12hrs with 5uM Bisabolene.

Figure 15 shows a mixed tissue culture treated for 12hrs with 100uM *trans*-β-Ocimene.

EXAMPLES

1. Growth curve analyses provide vital information on a cell lines growth pattern over a period of time *in vitro*.

The cell death inducing properties of ethanolic extracted *C. molmol* and hexane extracted Haddi on the S180 murine sarcoma cell line and the human fibrosarcoma HT1080 cell line were tested. Results from a previous experiments had indicated that a 667 ug/ml solution of both extracts was potent as a cell-death-inducing agent. A stock cell suspension with a cell density of $5x10^4$ cells/ml was freshly produced from an existing cell culture. Forty-eight flasks were labeled and seeded with 5ml of this cell suspension - 2 control/untreated, 2 ethanolic *C. molmol* and 2 hexane Haddi flasks for each day of the 8 day experiment. All flasks were incubated at 37°C and 5% $CO_2$. On day 4 the medium was changed in all remaining flasks. Untreated medium was used to replenish the control flasks while medium with an extract concentration of 667 ug/ml was used to replenish the relevant treatment flasks. Day 4 was chosen as the treatment day because this is the first day of the exponential growth phase of the growth curve. Treatment at this point in the growth curve would serve to fully demonstrate the ability of ethanolic *C. molmol* and hexane Haddi to induce cell death in this transformed cell line. Similar protocols were used to grow HT1080, a human fibrosarcoma cell line. In this instance growth was slower and cells were treated with extract when confluent at day 7 (See Fig.1).

Daily counts were carried out at the same time each day following standard protocol. Briefly, all medium was removed by pipetting. Flasks were then washed with 2ml PBS twice to remove as much cell debris as possible from the flasks (this would aid in cell counting). A final wash was carried out using 1ml Tris-EDTA, this was a very quick wash just to neutralise any remaining medium. All liquid was then carefully removed by pipetting before a further 1ml of Tris-EDTA was added to the flasks. These flasks were incubated at 37°C and 5% $CO_2$ for 2-5 minutes to ensure all cells had become detached from the flask. From day 4 onwards 2ml of Tris-EDTA was used to ease cell counting as the cell number had increased significantly. This cell suspension was mixed by gentle pipetting to ensure homogeneity and a sample then placed in a hemocytometer and cells were counted under a light microscope. In all, 4 counts were taken from each flask. Mean values were calculated and used to plot the growth curves (Figures 1 and 2).

Figure 2. clearly shows, S 180 cells display a classical sigmoidal growth curve. Cell density increases very slowly between day 0 and day 3 from $5x10^4$ cells/ml to $21.1x10^4$ cells/ml. Day 3 to day 6 is the exponential growth phase when cell density increases 10-fold to $240x10^4$ cells/ml, with a doubling time of approximately 24hrs during this phase. A plateau phase is observed between days 6 and 7 where cell number remains relatively constant and then begins to fall at day 8. This decrease in cell number is due to space and nutrient depletion in the cell culture.

Cells treated with hexane Haddi extract behave normally until day 4 (day of treatment). Day 5 sees a dramatic decrease in cell number to $1.8x10^4$ cells/ml from $68.6x10^4$ cells/ml on day 4. Cell number continues to decrease steadily reaching zero on day 8. This clearly demonstrates the ability of hexane extracted Haddi to induce cell death in rapidly growing transformed cell lines as the cells would normally be expected to continue growing until day 6 (see control curve). Ethanolic *C. molmol* is also seen to be a potent inducer of cell death in S180 cells. Again, up to day 4 the S180 cells follow the typical growth pattern. Following treatment on day 4 the cell number falls from $47.9x10^4$ cells/ml (day 4) to $12.8x10^4$ cells/ml on day 5. The decrease in cell number occurs at a slower rate than with hexane Haddi treatment.

Figure 1 shows similar response from human HT1080 fibrosarcoma cells following the addition of 667 ug/ml of ethanolic extracted C. *molmol* and hexane extracted Haddi to the growth medium on day 7. Untreated cells continue growing well up to day 13, whilst both *C. molmol* and Haddi treated cell lines decline rapidly in number from day 7.

2. MTT Assay for S180 sarcoma and HT1080 human fibrosarcoma cell lines treated with ethanolic *C. molmol* and hexane Haddi extracts

The MTT assay is a powerful, quantitative and highly reliable colourimetric assay that measures the viability of a cell population. The mitochondrial enzymes of viable cells are capable of metabolising 3-(4,5-dimethylthiazol-2-yl)-2,5-diphenyl tetrazolium bromide (MTT) from a yellow water-soluble compound to a water-insoluble purple formazan product - this is the chemical basis of the assay. The amount of formazan produced is directly proportional to the number of viable cells in the test population. The formazan product formed can be dissolved in dimethyl sulfoxide (DMSO), which enables quantification of the product to be obtained via spectrophotometry. A population containing largely viable cells will produce high levels of the formazan product and will therefore give a deep purple solution upon dissolution with DMSO, while a cell population that has mostly dead cells will produce a colourless solution. The amount of light absorbed (at a wavelength of 575nm) can be converted to % cytotoxicity by using the following calculation:

$$\frac{\text{[control absorption – test absorption]}}{\text{control absorption}} \times 100 = \% \text{ cytotoxicity}$$

Experimental Protocol

Two transformed cell lines were chosen for MTT analysis, S180 murine sarcoma cells and HT1080 human fibrosarcoma cells. Cells were seeded in 96-well microtitre plates at a density of $2x10^5$ cells/ml and incubated at 37°C and 5% $CO_2$ for 24 hours prior to treatment. 200ul PBS was added to the wells on the outer edges of the plate to reduce evaporation loss from the wells containing cells.

In this study we tested ethanolic C. *molmol* extract and hexane Haddi extract for cytotoxic activity against transformed cells. Extracts were added to medium for each cell line to give a treatment of 1333ug/ml. Serial dilutions were carried out to enable a range of progressively more dilute concentrations of extract to be tested. The same procedure was followed to make solutions of staurosporine and ethanol of the same concentrations. Staurosporine, an agent with well-established cytotoxic properties, was incorporated into the study to provide a benchmark to compare the cytotoxicity of the extracts against. The ethanol control was carried out because the extracts needed to be dissolved in ethanol prior to introduction into the medium, as the extracts contain mostly organic molecules that would not dissolve in medium alone - this control ensures that it is not the ethanol causing any observed cell death.

100ul of each concentration of each extract/control were added to 3 separate wells in a labeled microtitre plate. In the untreated control wells 100ul of medium was added - all wells having a total final volume of 200ul. 3 identical replicates of this were carried out for testing after 24, 48 and 72 hours. All plates were incubated at 37°C and 5% $CO_2$ until reading.

After the required length of time (i.e. 24, 48 or 72 hours) 20ul MTT solution (5mg/ml) was added to each well (excluding wells containing only PBS). Plates were then returned to the incubator (under the same conditions) for a further 4 hours. MTT is photosensitive so all plates were covered in foil to minimise exposure to light. After 4 hours all medium from the wells was pipetted out (care was taken to avoid disturbing the formazan crystals) and 200ul DMSO was added to each well. Careful mixing with a pipette ensured thorough dissolution. All plates were then read on a spectrophotometer for absorption of light at 575nm. This was done immediately after addition of DMSO. Results are seen in Figures 3 to 6.

The same method was also employed to test the impact of identified components of the Haddi extracts. Thus, *trans*-β-Ocimene and γ-Bisabolene both major components of the Haddi extract were tested using the MTTassay to estimate their impact on tumour cell cytotoxicity and viability, see Figures 7 to 10.

The cytotoxic effects of ethanol extracted *C. molmol* and hexane extracted Haddi on S180 fibrosarcoma and HT1080 human fibrosarcoma cell lines are shown in Figures 3 to 6. Both *C. molmol* and Haddi extracts produce a dose dependent increase in cytotoxicity with time. In general the effects of *C. molmol* extract were found to be more gradual than that of Haddi extract. *C. molmol* extract shows a gradual increase in cell death after 24 hours, whilst Haddi extract results in a more immediate cell death starting at lower doses.

The cytotoxic effects of *trans*-β-Ocimene and γ-Bisabolene on S180 and HT1080 tumour cell lines is shown in Figures 7 to 10.

The cytotoxic effects of trans-β-ocimene and γ-bisabolene were also tested on MM6 cells (monocyte leukaemia cell line) and A375 cells. The results are shown in Tables 5 to 8 below.

Table 1. Determination of Cytotoxicity of *trans*-β-Ocimene after 24, 48 and 72 hours on S180 Cells

| | % Cytotoxicity | | |
|---|---|---|---|
| **Concentration (mM)** | 24 hours | 48 hours | 72 hours |
| **250** | 92± 0 | 95 ± 0 | 95 ± 0 |
| **200** | 87± 3 | 95 ± 1 | 95 ± 0 |
| **150** | 82± 7 | 87 ± 5 | 87 ± 5 |
| **100** | 69 ± 11 | 82 ± 7 | 88 ± 4 |
| **50** | 19± 3 | 28± 4 | 22 ± 5 |
| • n = 3 replicates of 3 | | | |

Table 2. Determination of Cytotoxicity of *trans*-β-Ocimene after 24, 48 and 72 hours on HT1080 Cells

| **Concentration (mM)** | **% Cytotoxicity** | | |
|---|---|---|---|
| | 24 hours | 48 hours | 72 hours |
| **250** | 67± 2 | 90 ± 0 | 90 ± 1 |
| **200** | 62± 2 | 90 ± 0 | 90 ± 1 |
| **150** | 43± 8 | 71 ± 10 | 64 ± 12 |
| **100** | 16± 6 | 33 ± 5 | 15 ± 3 |
| **50** | 1± 5 | 0 ± 2 | -5 ± 3 |
| * n = 3 replicates of 3 | | | |

Table 3. Determination of Cytotoxicity of γ-Bisabolene after 24, 48 and 72 hours on S180 Cells

| | **% Cytotoxicity** | | |
|---|---|---|---|
| **Concentration (mM)** | 24 hours | 48 hours | 72 hours |
| **250** | 89±0 | 89±1 | 86±1 |
| **200** | 90±0 | 93±1 | 89±1 |
| **150** | 92±0 | 93±1 | 91±1 |
| **100** | 93±0 | 95±0 | 94±0 |
| **50** | 93± 1 | 95±0 | 94±0 |
| * n = 3 replicates of 3 | | | |

Table 4. Determination of Cytotoxicity of γ-Bisabolene after 24, 48 and 72 hours on HT1080 Cells

| | **% Cytotoxicity** | | |
|---|---|---|---|
| **Concentration (mM)** | 24 hours | 48 hours | 72 hours |
| **250** | 78± 2 | 89 ± 1 | 81 ± 2 |
| **200** | 87± 1 | 92 ± 1 | 88 ± 1 |
| **150** | 87± 1 | 93 ± 0 | 90 ± 0 |
| **100** | 90± 0 | 94 ± 1 | 91 ± 0 |
| **50** | 90± 0 | 94 ± 0 | 92 ± 0 |
| * n = 3 replicates of 3 | | | |

It is clear from the data that both *trans*-β-Ocimene and γ-Bisabolene are cytotoxic to S180 fibrosarcoma cells and human tumour cells grown in culture and represent anti-tumour drugs. Ocimene results in almost 100% tumour cell death at concentrations above 200mM and Bisabolene is potent at concentrations around 50 mM. Both drugs induce apoptosis (see following section). Ocimene is listed as a flavour and is permitted as food additive by the US Food and Drug Administration and is non toxic to mammals.

Table 5. Determination of Cytotoxicity of trans-β-ocimene after 24, 48 and 72 hours of MM6 cells (monocyte leukaemic cell line)

| | %Cytotoxicity | | |
|---|---|---|---|
| Concentration (μM) | 24 hours | 48 hours | 72 hours |
| 368 | 57 | 67 | 67 |
| 184 | 46 | 57 | 60 |
| 92 | 32 | 24 | 12 |
| 42 | 15 | -30 | -18 |

Table 6. Determination of Cytoxicity of trans-β-ocimene after 24, 48 and 72 hours on A375 cells (a human skin melanoma cell line)

| %Cytoxicity | | | |
|---|---|---|---|
| Concentration (μM) | 24 hours | 48 hours | 72 hours |
| 368 | 29 | 35 | 31 |
| 184 | 7 | 8 | -10 |
| 92 | -11 | -25 | -31 |
| 46 | 7 | -2 | -33 |

Table 7. Determination of Cytoxicity of γ-Bisabolene after 24, 48 and 72 hours on MM6 cells

| %Cytoxicity | | | |
|---|---|---|---|
| Concentration (μM) | 24 hours | 48 hours | 72 hours |
| 10 | 45 | 67 | 78 |
| 2.5 | 51 | 70 | 78 |
| 0.25 | 49 | 68 | 80 |
| 0.13 | 3 | -37 | 13 |

Table 8. Determination of Cytoxicity of γ-Bisabolene after 24, 48 and 72 hours on A375 cells

| %Cytoxicity | | | |
|---|---|---|---|
| Concentration (μM) | 24 hours | 48 hours | 72 hours |
| 10 | 23 | 64 | 77 |
| 2.5 | 15 | 55 | 71 |
| 0.25 | 10 | 7 | 50 |
| 0.13 | 15 | 5 | 13 |

The chemical γ-Bisabolene is a potent anti-tumour agent, inducing high cell death at concentrations as low as 0.25μM for both tumour cell types.

Annexin V Bioassay of apoptosis in HT1080 Fibrosarcoma cells treated with ethanolic *C. molmol* and hexane Haddi

The Annexin V bioassay is used to distinguish between cells dying via the apoptotic route and cells dying by necrosis. Knowing how ocimene and bisabolene cause cell death is crucial. If the compounds caused necrotic death they would have very little potential as therapeutic agents. This is because necrotic cells burst and release their content into the surroundings, thus causing an inflammatory response that is potentially harmful to neighbouring, non-target cells. Apoptosis on the other hand produces a natural discrete form of cell suicide, which does not cause inflammation.

The Annexin V bioassay utilises an early change in the plasma membrane of apoptotic cells during which phosphatidylserine (PS), a membrane bound phospholipid, is translocated from the inner surface of the membrane bi-layer to the outer surface - thus bringing the PS into contact with the environment. This change occurs before membrane blebbing and far earlier than DNA fragmentation thus making it an ideal marker for early apoptotic cells. Annexin V is a $Ca^{2}$+-dependent phospholipid binding protein with a high affinity for PS. In this study the Annexin V was coupled to a fluorescent marker called FITC, to generate an Annexin V-FITC conjugate. FITC fluoresces with an intense green/yellow colour when viewed via fluorescent microscopy. Thus, early apoptotic cells will bind Annexin V-FITC on their plasma membranes and fluoresce green/yellow, whereas late apoptotic cells will fluoresce green/yellow in the cytoplasm as well as at the membrane.

To identify necrotic cells another dying agent, propidium iodide (PI) is used. PI is a DNA intercalating agent that stains the chromatin of cells and fluoresces red/orange. As PI is not able to cross an intact plasma membrane it only stains the chromatin of cells that have damaged membranes, i.e. necrotic or late-apoptotic cells that become leaky.

HT1080 cells were grown on cover-slips in 12-well plates at a cell density of $1x10^6$ cells/ml at 37°C and 5% $CO_2$. Once established, these cultures were treated with a 0.2% solution of extract (2 μl extract in 2 μl ethanol and 996

μl medium) for 24 hours.

An untreated control was also carried out under the same conditions.

Annexin V-FITC and PI staining was carried out according to the manufacturers protocol (Oncogene™ Research Products, Boston USA). All growth medium was removed and cells were washed once with 1ml PBS and 500 μl binding buffer added to the cells. 10 μl media binding agent (containing $CaCl_2$ as a source of calcium, as the Annexin V-PS binding is $Ca^{2+}$ dependent) and 1.25μl Annexin V-FITC was added to the wells. This was then incubated for 15 minutes at room temperature (~22°C) in the dark, as Annexin V-FITC is photosensitive. All media was removed by pipetting and 500 μl of cold binding buffer added to the cells. 10 μl of propidium iodide solution was added.

The cover-slip was removed from the well and mounted, cells facing down, on a glass slide using Vectashield™ mounting medium. Cells were then viewed immediately at x25 magnification on a fluorescent microscope using FITC and rhodamine filters.

Examples

The Annexin V test below shows the induction of apoptotic cell death in HT1080 human fibrosarcoma cells treated with 5uM Bisabolene over 12 hours. Cells fluorescing green (shown as white in the Figure) indicate entry into apoptosis, a programmed form of cell death (see Figure 11).

Similar results were obtained with S180 fibrosarcoma cells and with HT1080 human fibrosarcoma cells treated with 100 uM Ocimene over 12 hours.

The individual chemicals tested to date represent major constituents of scented Myrrh or Haddi. Much work remains to be done on isolating and testing the major constituents of Myrrh or *molmol.* In this respect particular attention will be given to testing the anti-tumour properties of Furanosesquiterpenoids some of which remain to be identified and characterised.

Normal Fibroblast Cells

Normal fibroblast cells grown in a control culture showed a very low normal occurrence of apoptotic cells, a mean of 3% of a total 400 cells counted, in contrast to 97% normal live cells. When normal fibroblast cells were grown in a medium containing 666 μg/ml ethanolic *C. molmol*, the production of apoptotic cells was much less than in S180 and HT1080 tumour cell lines, increasing from 4.5% apoptotic cells after 6 hours of incubation to 32% after 24 hours. A similar observation was noted when cells were grown in media containing 234 μg/ml ethanolic *C. molmol,* though lower percentages of apoptotic cells were observed, increasing from 6.25% after 6 hours of incubation to 19.25% after 24 hours of incubation. See figure 12.

In a separate experiment normal fibroblast cells grown in a control culture also showed a slight normal occurrence of apoptotic cells, a mean of 2.25% of a total 400 cells counted, in contrast to 97.75% normal live cells. When normal fibroblast cells were grown in a medium containing 666 μg/ml hexane Haddi extract, the production of apoptotic cells was much less than that obtained in S180 and HT1080 tumour cell lines, increasing from 16% apoptotic cells after 6 hours of incubation to only 37% after 24 hours. A similar observation was noted when cells grown in media containing 201 μg/ml hexane Haddi extract, though lower percentages of apoptotic cells were observed, increasing from 11% after 6 hours of incubation to only 25.5% after 24 hours of incubation. See figure 13.

It is noted that hexane extracted Haddi performed slightly better in producing apoptosis in S180 tumour cells than ethanolic *C. molmol.* Ethanolic *C. molmol* extract and hexane Haddi extract treatments produced very similar results in terms of the percentages of apoptotic cells in HT1080 human fibrosarcoma cells. The difference was not significant.

It is clear however that both extracts produce a differentially higher level of apoptosis in the transformed fibrosarcoma cells than in the normal fibrocytes. This means that at suitable doses these extracts are differentially active against tumour cells.

Similar results have been obtained in terms of a differential response to Ocimene and Bisabolene. In addition when mixed cultures containing fibroblast cells and tumour cells are treated, the normal fibroblasts appear to survive while the tumour cells go into apoptosis.

The results of such an experiment are shown in Figures 14, wherein a mixed tissue culture has been treated for 12hrs with 5uM Bisabolene. Note the flattened fibrocytes survive whilst tumour cells round off as apoptotic bodies. In Figure 15 a mixed tissue culture has been treated for 12hrs with 100uM Ocimene. Note fibrocytes spread out and survive whilst tumour cells round off to form apoptotic bodies.

## Claims

**1.** Use of a compound isolated from a plant, or synthesised, in the manufacture of a medicament for the treatment or

prophylaxis of cancer, wherein the compound is selected from:

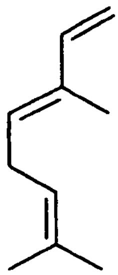

trans-β-Ocimene; and

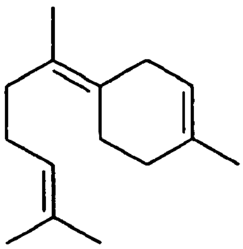

γ-bisabolene

2. The use of claim 1, wherein the compound is trans-β-ocimene.

3. The use of claim 1, wherein the compound is γ-bisabolene.

4. The use of claim 1, wherein the compound is used at a concentration of between 0.2μM and 100mM.

5. A pharmaceutical composition comprising:

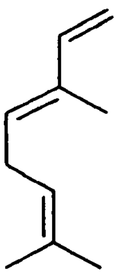

trans-β-Ocimene,

isolated from a plant, or synthesised, in combination with a pharmaceutically acceptable excipient, carrier, adjuvant or vehicle.

6. The pharmaceutical composition according to claim 5, which is formulated so that a single unit dose of the composition

provides trans-β-ocimene at a concentration of between 0.2μM and 100mM per unit dose.

7. The use of claim 1, wherein the cancer is breast cancer, ovarian cancer, prostate cancer, colon cancer, lung cancer, pancreatic cancer, bowel cancer, melanoma, testicular cancer, cervical cancer, fibrosarcoma, squamous cell carcinoma, leukaemia, astrocytoma or glioma.

8. Use of a *Commiphora guidotti* extract in the manufacture of a medicament for the treatment or prophylaxis of cancer.

**Patentansprüche**

1. Verwendung einer aus einer Pflanze isolierten oder synthetisierten Verbindung zur Herstellung eines Medikaments zur Behandlung oder Prophylaxe von Krebs, wobei die Verbindung ausgewählt ist aus:

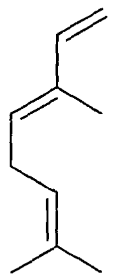

trans-β-Ocimen; und

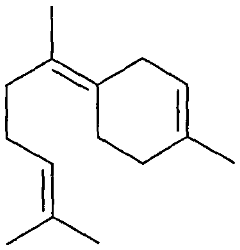

γ-Bisabolen

2. Verwendung nach Anspruch 1, wobei die Verbindung trans-β-Ocimen ist.

3. Verwendung nach Anspruch 1, wobei die Verbindung γ-Bisabolen ist.

4. Verwendung nach Anspruch 1, wobei die Verbindung in einer Konzentration zwischen 0,2 μM und 100 mM verwendet wird.

5. Pharmazeutische Zusammensetzung, umfassend

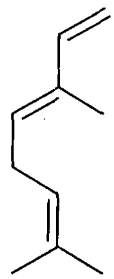

trans-β-Ocimen

isoliert aus einer Pflanze oder synthetisiert, in Kombination mit einem pharmazeutisch verträglichen Hilfsstoff, Träger, Adjuvans oder Vehikel.

6. Pharmazeutische Zusammensetzung nach Anspruch 5, welche so formuliert ist, dass eine Einzeldosis der Zusammensetzung trans-β-Ocimen in einer Konzentration zwischen 0,2 μM und 100 mM pro Dosierungseinheit vorsieht.

7. Verwendung nach Anspruch 1, wobei der Krebs Brustkrebs, Ovarialkrebs, Prostatakrebs, Dickdarmkrebs, Lungenkrebs, Pankreaskrebs, Darmkrebs, Melanom, Hodenkrebs, Gebärmutterhalskrebs, Fibrosarkom, Plattenepithelkarzinom, Leukämie, Astrozytom oder Gliom ist.

8. Verwendung eines *Commiphora guidotti*-Extrakts zur Herstellung eines Medikaments zur Behandlung oder Prophylaxe von Krebs.

**Revendications**

1. Utilisation d'un composé isolé à partir d'une plante, ou synthétisé, dans la fabrication d'un médicament pour le traitement ou la prophylaxie du cancer, où le composé est choisi parmi :

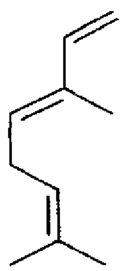

le trans-β-ocimène ; et

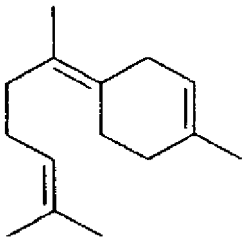

le γ-bisabolène

2. Utilisation selon la revendication 1 où le composé est le trans-β-ocimène.

3. Utilisation selon la revendication 1 où le composé est le γ-bisabolène.

4. Utilisation selon la revendication 1 où le composé est utilisé à une concentration entre 0,2 μM et 100 mM.

5. Composition pharmaceutique comprenant :

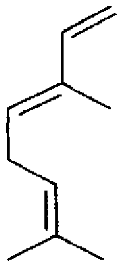

du trans-β-ocimène

isolé à partir d'une plante, ou synthétisé, en combinaison avec un excipient, vecteur, adjuvant ou véhicule pharmaceutiquement acceptable.

6. Composition pharmaceutique selon la revendication 5 qui est formulée de sorte qu'une seule dose unitaire de la composition fournit le trans-β-ocimène à une concentration entre 0,2 μM et 100 mM par dose unitaire.

7. Utilisation selon la revendication 1 où le cancer est le cancer du sein, le cancer ovarien, le cancer de la prostate, le cancer du côlon, le cancer du poumon, le cancer pancréatique, la cancer de l'intestin, le mélanome, le cancer testiculaire, le cancer cervical, le fibrosarcome, le carcinome épidermoïde, la leucémie, l'astrocytome ou le gliome.

8. Utilisation d'un extrait de *Commiphora guidotti* dans la fabrication d'un médicament pour le traitement ou la prophylaxie du cancer.

Figure 1

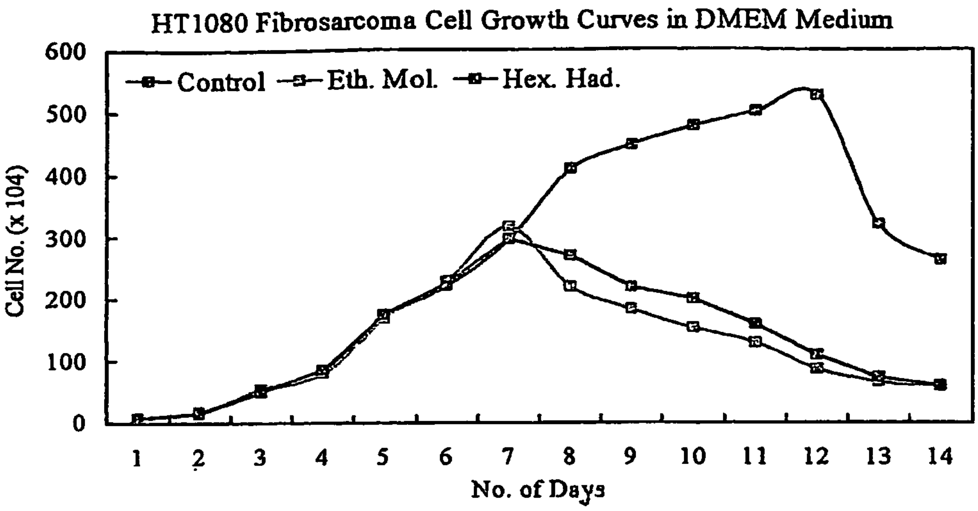


Figure 2

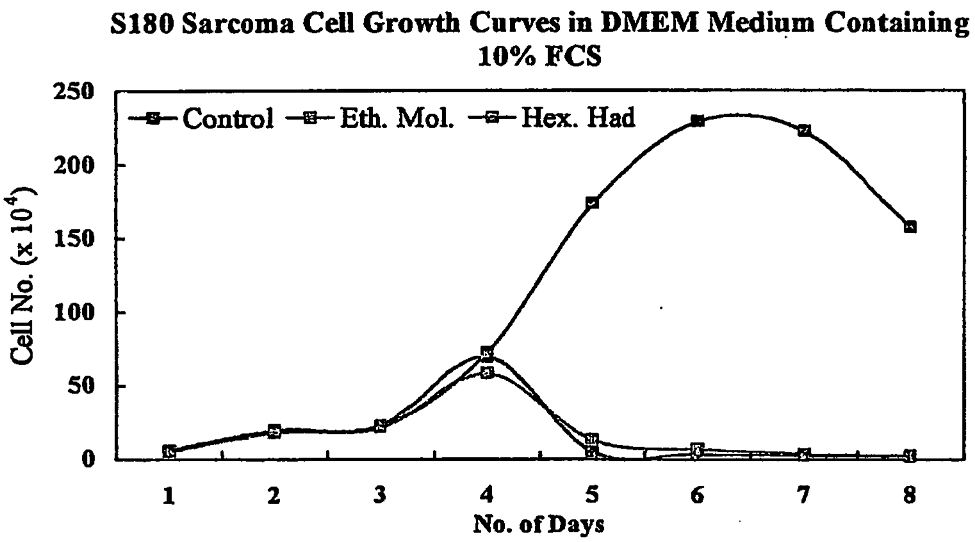

Figure 3

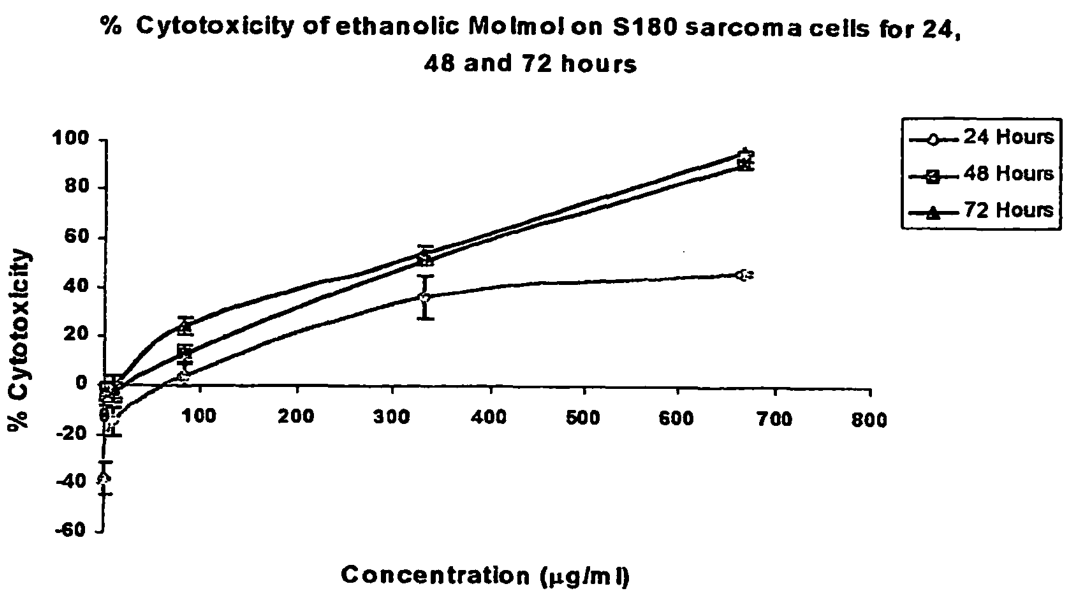


Figure 4

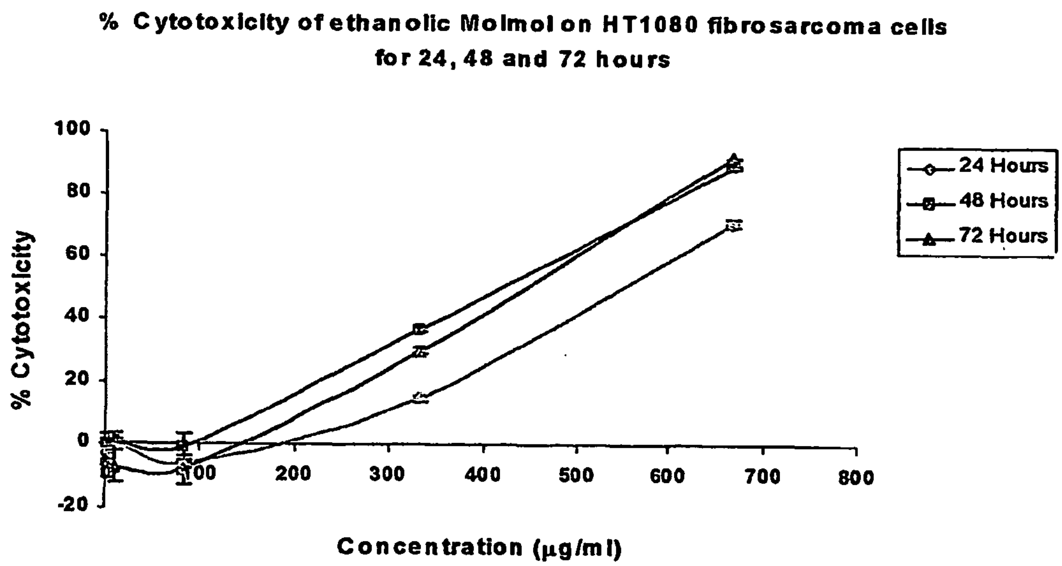

Figure 5

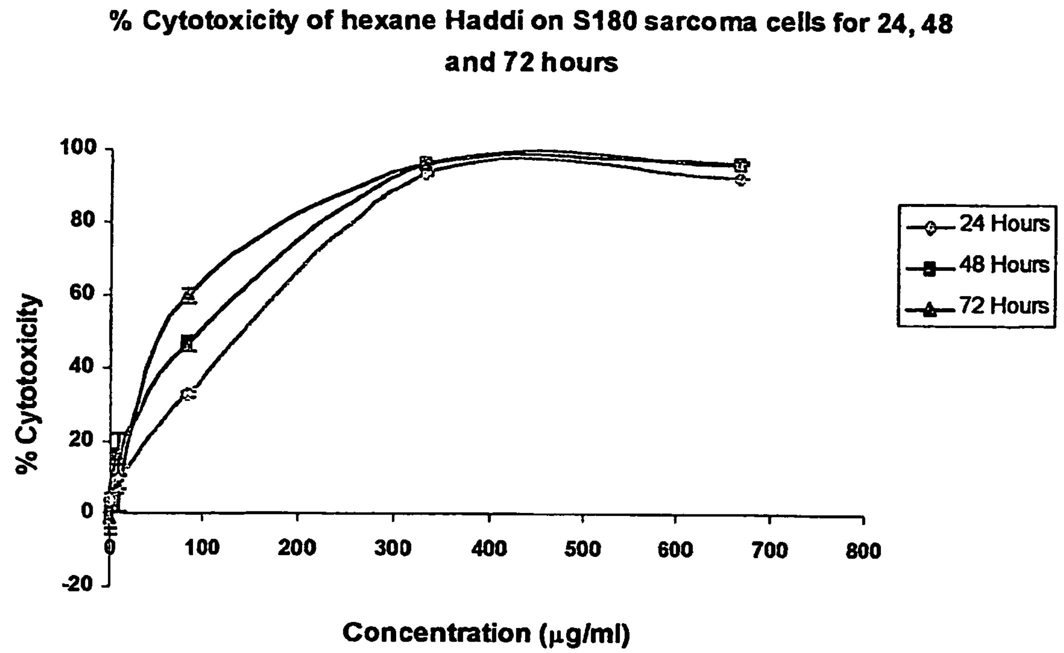


Figure 6

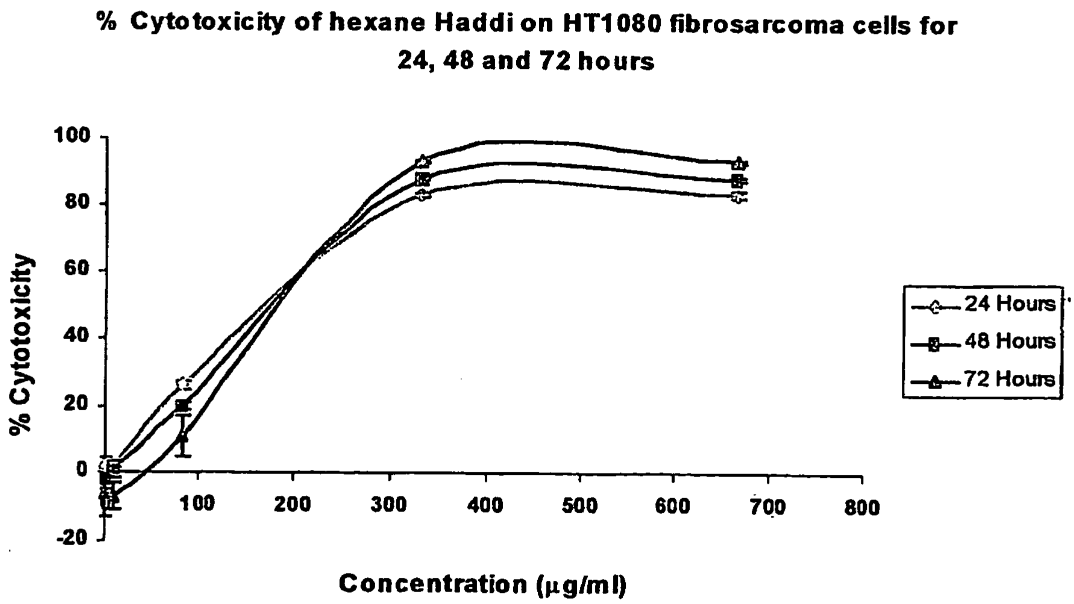

Figure 7

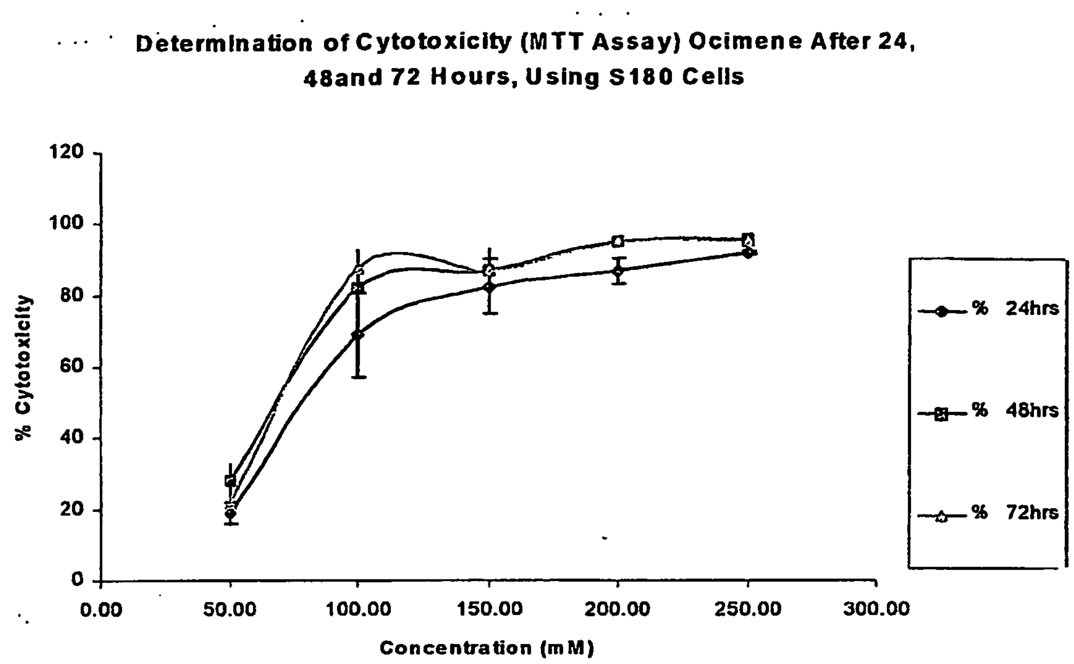


Figure 8

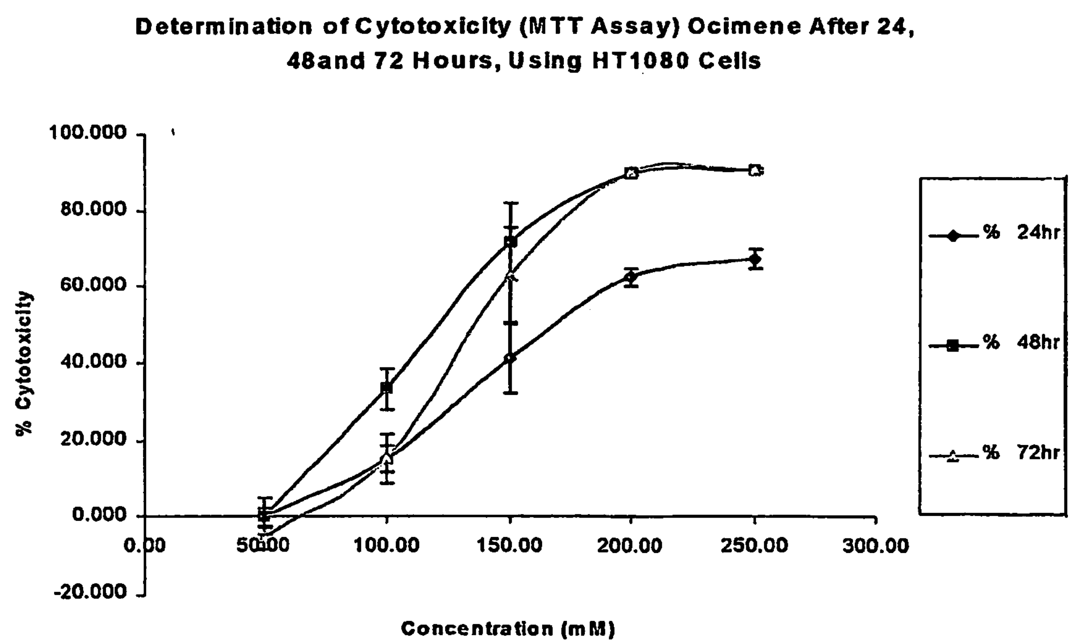

Figure 9

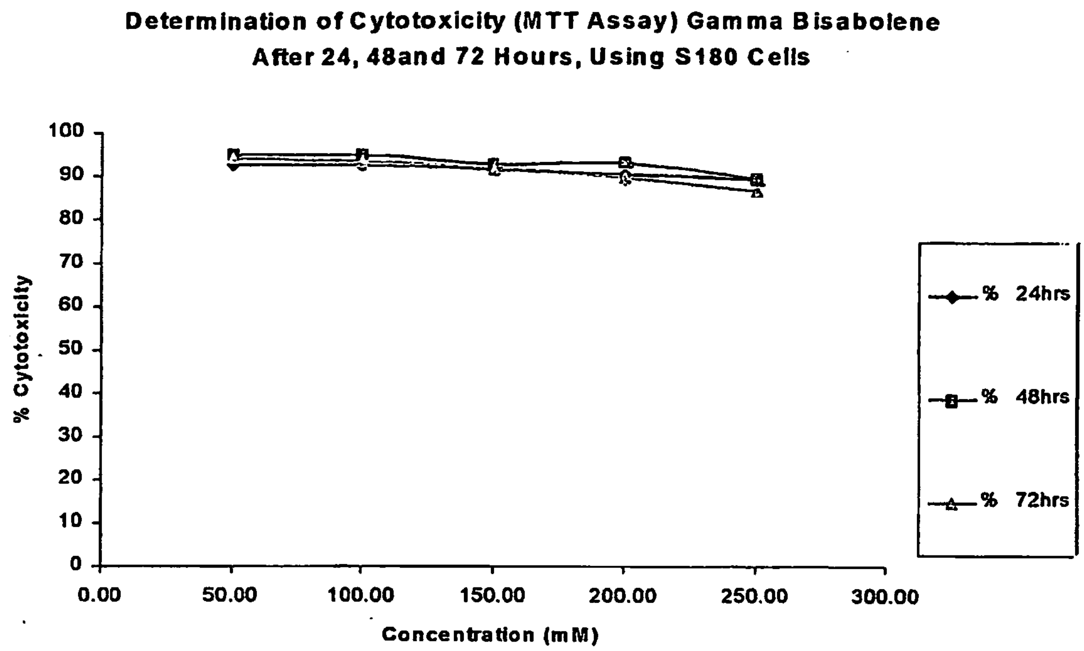


Figure 10

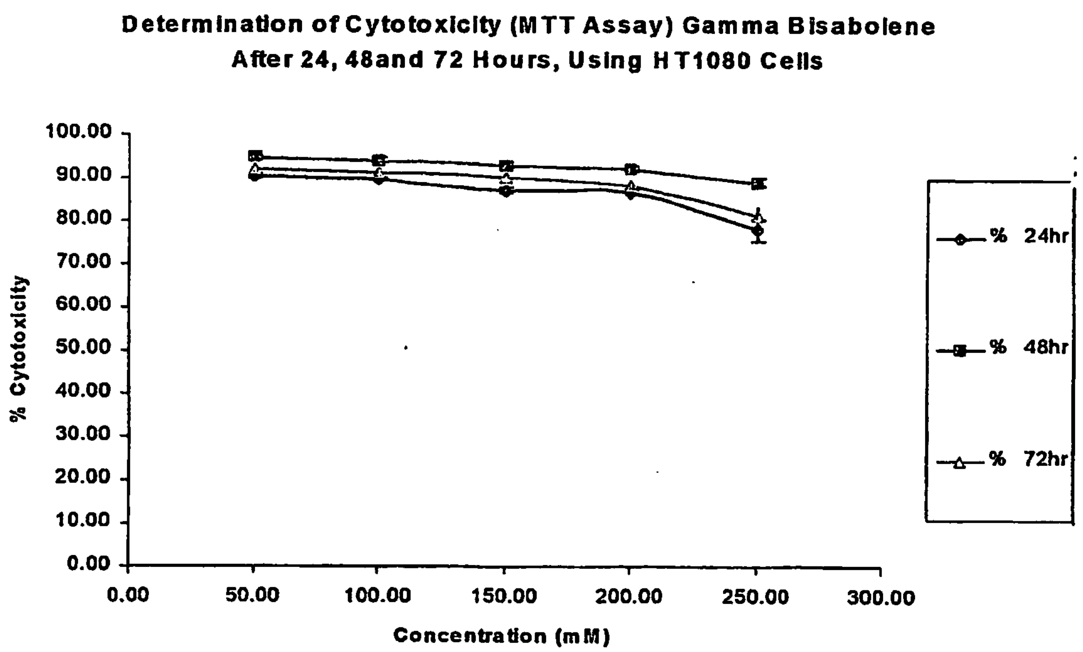

Figure 11

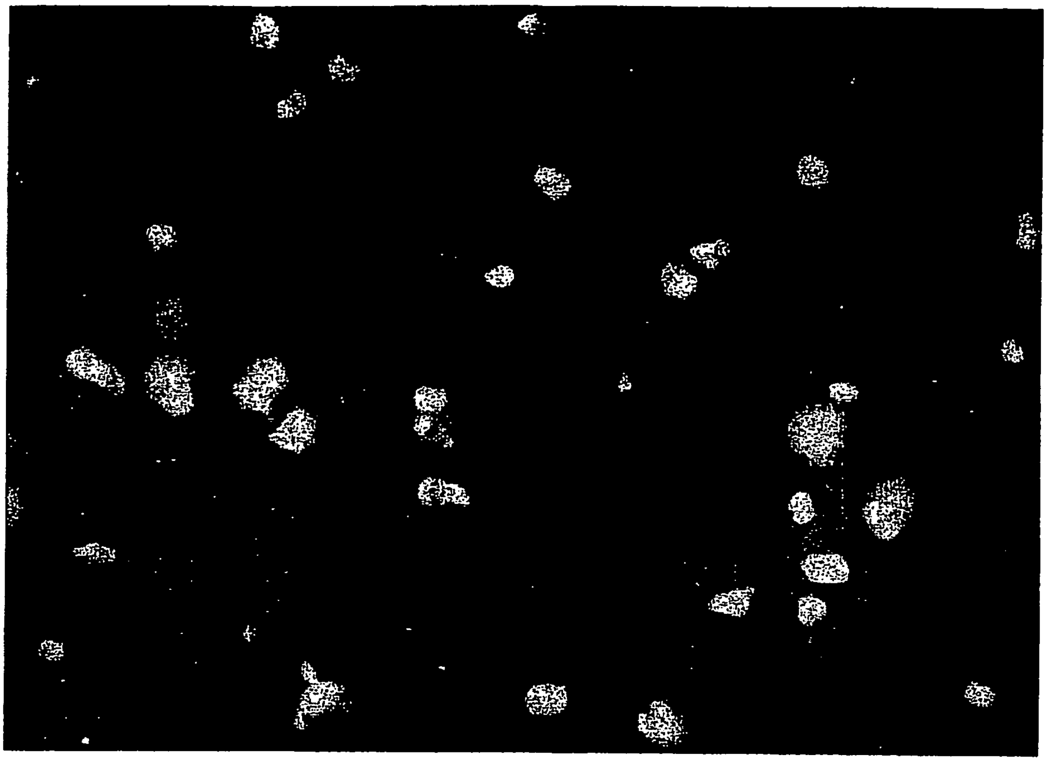

Figure 12

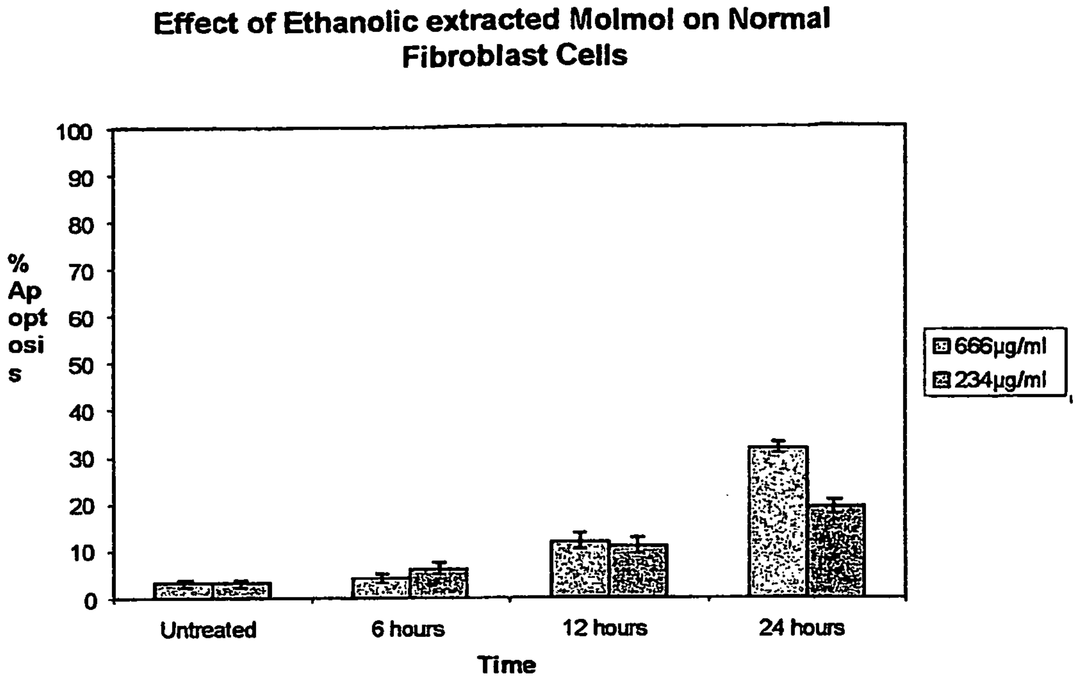


Figure 13

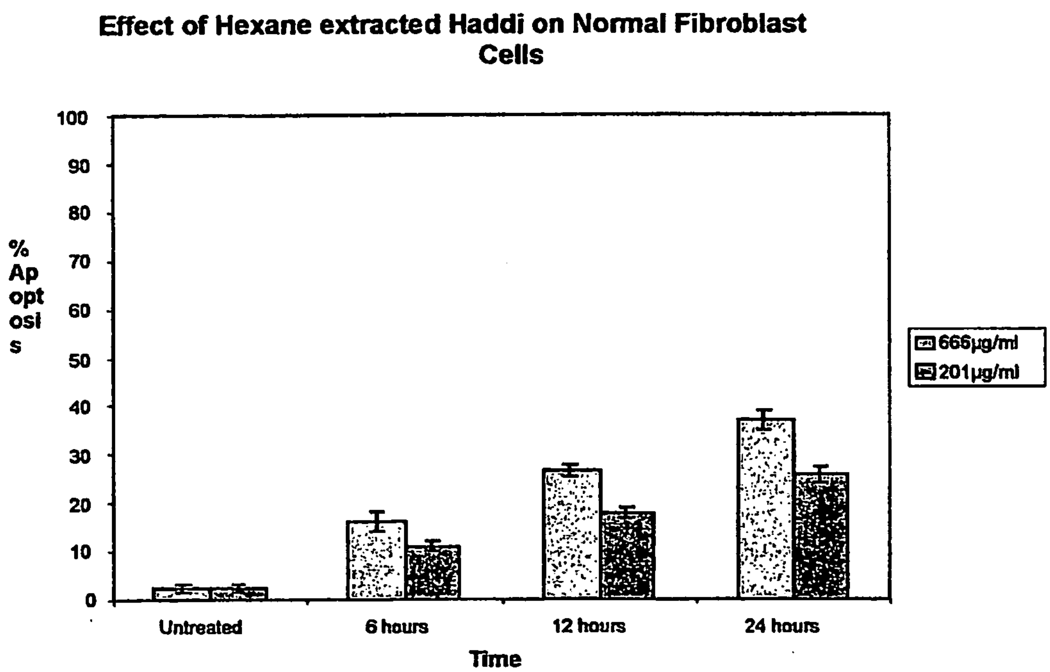

Figure 14

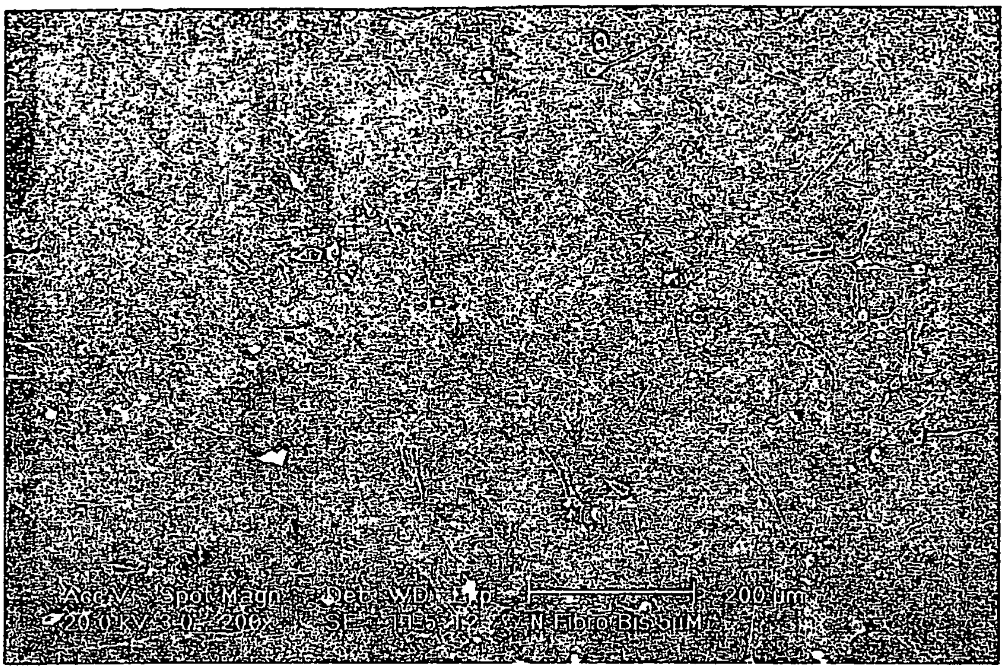


Figure 15

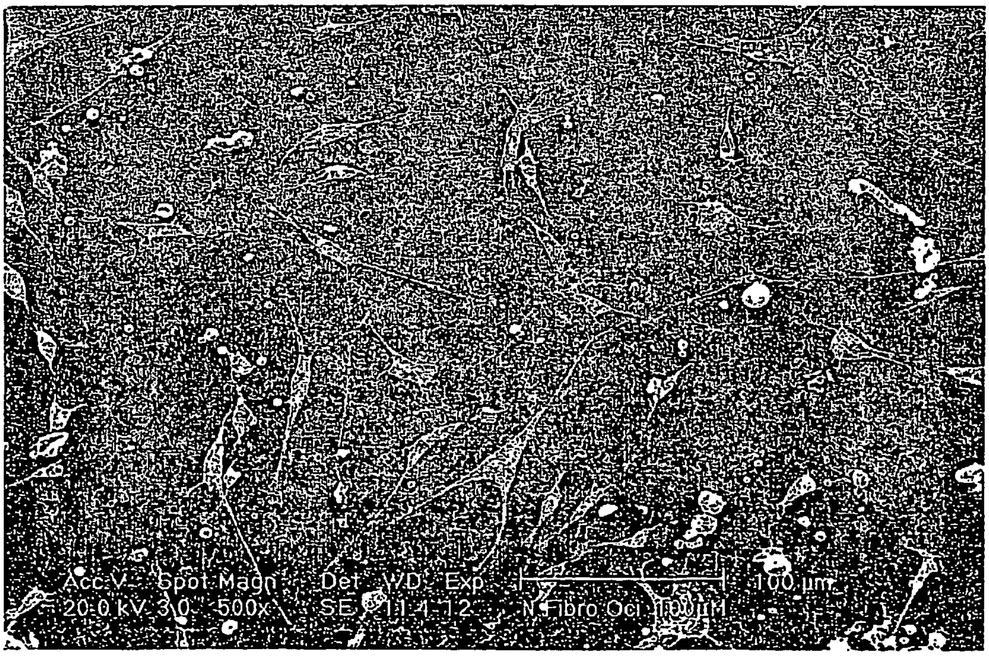

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0050053 A
- US 6486706 A
- US 5602184 A
- US 20020155522 A
- WO 0180868 A


### Non-patent literature cited in the description


- **Al Harbi et al.** *Experimental Chemotherapy,* 1994, vol. 40, 337-347
- **Qureshi ; Al Harbi et al.** *Cancer Chemotherapy and Pharmacology,* 1993, vol. 33, 130-138
- **Rao et al.** *J. Ethnopharmacology,* 2001, vol. 76, 151-154
- **Tatman et al.** *Cancer Letters,* 2002, vol. 175, 129-139
- **Leal et al.** *J. Agric. Food. Chem.,* 2003, vol. 51, 2520-2525
- **Saleh et al.** *Pharm. Pharmacol. Lett.,* 1998, vol. 8, 97-99
- **Itokawa et al.** *Chem. Pharm. Bull.,* 1985, vol. 33, 3488-3492